# EUROPEAN PATENT SPECIFICATION

(11) **EP 2 931 222 B1**
(45) Date of publication and mention of the grant of the patent: **15.02.2017**
(21) Application number: 12806319.5
(22) Date of filing: 14.12.2012
(51) Int. Cl.: A61K 8/26, A61K 8/36, A61Q 11/00, A61K 8/81, A61K 8/02, A61K 8/22, A61K 8/891

(54) **ABRASIVE COATINGS FOR PEROXIDE-CONTAINING COMPOSITIONS**
ABRASIVE BESCHICHTUNGEN FÜR PEROXIDHALTIGE ZUSAMMENSETZUNGEN
REVÊTEMENTS ABRASIFS POUR COMPOSITIONS CONTENANT DU PEROXYDE

(43) Date of publication of application: 21.10.2015
(73) Proprietor: Colgate-Palmolive Company, New York, NY 10022 (US)
(72) Inventor: MALONEY, Venda Porter, Piscataway, New Jersey 08854 (US); CHOPRA, Suman, Monroe, New Jersey 08831 (US); MANDADI, Prakasarao, Flemington, New Jersey 08822 (US)
(74) Representative: Daniels, Jeffrey Nicholas
(86) International application number: PCT/US2012/069885
(87) International publication number: WO 2014/092736

(56) References cited:
- WO-A1-02/085319
- FR-A1- 2 785 534
- US-A- 4 098 878
- US-A- 4 971 782
- US-A1- 2006 193 791
- US-A1- 2012 301 522

## Description

### BACKGROUND

Dentifrice formulations comprising peroxide are known and useful for cleaning and whitening teeth. The peroxide can bleach the teeth, remove stains, and kill cariogenic bacteria. However, peroxide compounds are highly reactive, and consequently difficult to formulate. Moreover, hydrogen peroxide can spontaneously decompose to form oxygen gas (O₂) and water, so that on storage, the dentifrice containers may bloat, burst or leak, and the remaining formulation will not have enough peroxide remaining to clean and whiten teeth effectively. Some initially comprise very high levels of peroxide, which decomposes over time, so that the exact amount of peroxide delivered on application is variable and largely depends on how long and under what conditions the dentifrice has been stored.

However, known whitening dentifrice compositions including peroxide may exhibit an unacceptable level of peroxide decomposition and loss of whitening efficacy as a result of being stored prior to sale or by the user.

There is thus a need for improved peroxide-containing whitening oral care compositions, for example dentifrice compositions, which exhibit improved cosmetic stability of the peroxide, and so are chemically stable for long-term storage and are suitable for everyday consumer use without significant loss of whitening efficacy. Embodiments of the present invention at least partly aim to meet these needs US2012301522 discloses peroxide-stabilised abrasive tooth whitening compositions.

### SUMMARY

Some embodiments of the present invention provide a single phase whitening oral care composition, which exhibits cosmetic chemical stability of the peroxide and physical stability of the composition, and so is chemically and physically stable for long-term storage and is suitable for everyday consumer use, and remains effective to clean and whiten teeth.

Accordingly, the invention provides an oral care composition comprising (i) a peroxide whitening agent, and (ii) an abrasive in the form of particles, wherein the particles are pre-coated with a coating layer comprising of a coating material selected from a substantially saturated C₂ - C₃₀ fatty acid, a monounsaturated C₁₂ - C₃₀ fatty acid, and a silicone polymer, or a combination of two or more thereof.

Optionally, the coating material has an average molecular weight of less than 500.

Optionally, the substantially saturated C₂ - C₃₀ fatty acid and the monounsaturated C₁₂ - C₃₀ fatty acid each have a carbon-carbon bond saturation of at least 90%, further optionally at least 94%.

Optionally, the substantially saturated C₂ - C₃₀ fatty acid comprises a substantially saturated C₁₂ - C₂₄ fatty acid, for example stearic acid.

Optionally, the monounsaturated C₁₂ - C₃₀ fatty acid comprises a monounsaturated C₁₆ - C₃₀ fatty acid, for example oleic acid.

Optionally, the silicone compound comprises a polydialkysiloxane, for example polydimethylsiloxane.

Optionally, the coating layer is nonionic.

Optionally, the coating layer has a minimum of a monolayer of the coating material, typically at least 10 Å.

Optionally, the coating layer comprises from 0.01 to 10 wt% of the weight of the coated abrasive particles, e.g., 0.05 to 10 wt%.

Optionally, the abrasive is selected from at least one of calcined alumina, silica, zirconium oxide, calcium pyrophosphate, dicalcium phosphate and precipitated calcium carbonate, or any mixture of two or more thereof.

Optionally, the pre-coated abrasive has an average particle size of from 1 to 20 microns.

Optionally, the pre-coated abrasive is present in an amount of from 5 wt % to 15 wt % based on the weight of the composition.

Optionally, the peroxide whitening agent is selected from hydrogen peroxide, crosslinked polyvinylpyrrolidone complexed with hydrogen peroxide, urea peroxide, calcium peroxide and sodium percarbonate, or any mixture of two or more thereof.

Optionally, the peroxide whitening agent comprises crosslinked polyvinvlpyrrolidone complexed with hydrogen peroxide, the abrasive comprises calcined alumina and the coating material comprises stearic acid.

Optionally, the crosslinked polyvinvlpyrrolidone thickening agent is present in an amount of from 3 wt % to 8 wt %, further optionally from 5 wt % to 7 wt %, based on the weight of the composition.

In some embodiments, the composition further comprises an ethylene oxide, propylene oxide block co-polymer of average molecular weight greater than 5000 Da, being substantially free of an ethylene oxide, propylene oxide block co-polymer of average molecular weight less than 5000 Da. Typically, the ethylene oxide, propylene oxide block co-polymer comprises (ethylene oxide)ₓ-(propylene oxide)_{y} wherein x is an integer of 80-150 and y is an integer 30-80. Optionally, the ethylene oxide, propylene oxide block co-polymer is present in an amount of from 5 wt % to 10 wt % based on the weight of the composition.

Optionally, the crosslinked polyvinylpyrrolidone complexed with hydrogen peroxide is present in an amount of from 3 wt % to 8 wt % based on the weight of the composition. Optionally, the whitening complex contains 10-30 wt% hydrogen peroxide and 5-15 wt% total nitrogen, based on the weight of the whitening complex. Optionally, the total amount of hydrogen peroxide is from 0.5 wt% to 3 wt% based on the weight of the composition.

In some embodiments, the composition further comprises polyethylene glycol of average molecular weight 400 to 800 Da. Optionally, the polyethylene glycol is present in an amount of from 5 wt % to 15 wt % based on the weight of the composition.

In some embodiments, the composition further comprises at least one humectant selected from glycerin and propylene glycol, or a mixture thereof. Optionally, the at least one humectant is present in an amount of from 35 wt % to 60 wt % based on the weight of the composition, further optionally from 30 wt % to 55 wt % based on the weight of the composition. In some embodiments, the composition comprises propylene glycol in an amount of from 15 wt % to 30 wt % based on the weight of the composition. In some embodiments, the composition comprises glycerin in an amount of from 15 wt % to 30 wt % based on the weight of the composition.

Optionally, the composition contains less than 3 wt% water based on the weight of the composition.

Optionally, the composition is a single phase composition.

In some embodiments, the composition is a toothpaste comprising a calcined alumina abrasive pre-coated with the coating layer.

In some embodiments, the composition further comprises an anionic surfactant in an amount of from 0.5 to 3 wt% based on the weight of the composition.

In the preferred embodiments of the invention, the oral care compositions are chemically and physically stable during long term storage and remain effective to clean and whiten teeth, with good cosmetic stability during manufacture and use of the compositions.

The inventors have unexpectedly found that an abrasive pre-coated with a thin layer of an organic carbon-containing or silicon-containing molecule can increase peroxide stability in dentifrice as compared to an uncoated abrasive particle.

By providing a coating material that is not reactive with peroxide when coated on an abrasive, the peroxide stability can be enhanced.

The invention also provides a method of tooth whitening comprising applying the composition of the invention to the surface of a mammalian tooth.

Further embodiments of the invention will be apparent from the detailed description and the examples.

### DETAILED DESCRIPTION

As used throughout, ranges are used as shorthand for describing each and every value that is within the range. Any value within the range can be selected as the terminus of the range.

In some embodiments, the present invention provides an oral care composition comprising (i) a peroxide whitening agent, and (ii) an abrasive in the form of particles, wherein the particles are pre-coated with a coating layer comprising a coating material selected from a substantially saturated C₂ - C₃₀ fatty acid, a monounsaturated C₁₂ - C₃₀ fatty acid, and a silicone polymer, or a combination of two or more thereof.

The oral care composition typically is a single phase composition, for example a toothpaste.

In some embodiments, the abrasive is selected from at least one of calcined alumina, silica, zirconium oxide, calcium phosphate, calcium pyrophosphate, dicalcium phosphate dicalcium orthophosphate, tricalcium phosphate, calcium polymetaphosphate, and precipitated calcium carbonate, or a combination of two or more thereof.

Typically, the pre-coated abrasive is present in an amount of from 5 wt % to 15 wt % based on the weight of the composition.

The average abrasive particle size is generally about 0.1 to about 30 microns, for example about 1 to about 20 microns or about 5 to about 15 microns. Optionally, the pre-coated abrasive has an average particle size of from 1 to 10 microns.

In some embodiments, the abrasive particles have an oil of absorption of from about 5g to about 250g/100g. In some embodiments, the abrasive particles have a surface area of from about 0.1 to 500 m²/g, more preferably 10 to 300 m²/g, further preferably 50 to 200 m²/g.

In some embodiments, the abrasive comprises alumina. In some embodiments, the abrasive comprises calcined alumina.

In some embodiments, the coating material has an average molecular weight of less than 500.

In some embodiments, the substantially saturated C₂ - C₃₀ fatty acid and the monounsaturated C₁₂ - C₃₀ fatty acid each have a carbon-carbon bond saturation of at least 90%, further optionally at least 94%. The coating material should not include a molecule having any more than a very minor level of conjugation, for example provided by an alkene, alkyne or other conjugated molecule, since such unsaturated bonds react with peroxide when coated onto an abrasive particle, leading to peroxide decomposition.

In some embodiments, the substantially saturated C₂ - C₃₀ fatty acid comprises a substantially saturated C₁₂ - C₂₄ fatty acid, for example stearic acid.

In some embodiments, the monounsaturated C₁₂ - C₃₀ fatty acid comprises a monounsaturated C₁₆ - C₃₀ fatty acid, for example oleic acid.

In some embodiments, the silicone polymer comprises a polydialkysiloxane, for example polydimethylsiloxane.

In some embodiments, the silicone polymer is selected from a silicone elastomer gel, stearoxytrimethylsilane (and) stearyl alcohol, alkylmethyl siloxane, alkymethyl siloxane colpolyol, polyphenylsilsesquioxane, trimethylsiloxysilicate, methyl silicone resin, and a crotonic acid/vinyl C8-12 isoalkyl esters/VA/BIS-vinyldimethicone crosspolymer.

In some embodiments, the coating layer is nonionic.

In some embodiments, the coating layer has a thickness of from a minimum of a monolayer of the coating material, typically at least 1 angstrom (Å). In some embodiments, the thickness of the coating layer is from about 1 Å to about 10 nm. In some embodiments, the thickness of the coating layer is from about 10 Å to about 1 nm. In some embodiments, the coating layer comprises from 0.01 to 10 wt% of the weight of the coated abrasive particles.

In some embodiments, the whitening agent may comprise any peroxide whitening agent, for example hydrogen peroxide or a source of bound hydrogen peroxide such as a PVP-H₂O₂ complex, urea peroxide, calcium peroxide or sodium percarbonate

In some embodiments, the crosslinked polyvinylpyrrolidone complexed with hydrogen peroxide is present in an amount of from 0.5 wt % to 16 wt % based on the weight of the composition.

Typically, the whitening complex contains 10-30 wt% hydrogen peroxide and 5-15 wt% total nitrogen, based on the weight of the whitening complex. In some embodiments, the total amount of hydrogen peroxide is from 0.05 wt% to 5 wt% based on the weight of the composition, e.g., 0.1 to 3 wt%, e.g. about 1 wt%, about 2 wt%, or about 3 wt%.

Typically, the whitening complex contains about 15-25%, for example about 17-22% of hydrogen peroxide by weight, and about 7-12% total nitrogen by weight; for example, having substantially the same specification as Polyplasdone® XL-10, e.g., Polyplasdone® XL-10F, e.g., available from International Specialty Products (Wayne, NJ).

In some embodiments, the composition includes a thickening system in which a thickening agent, such as crosslinked polyvinvlpyrrolidone, is provided which thickens the composition to enable the composition to be extruded by a user from a container such as a tube to enable the composition to be used as a toothpaste or gel.

In some embodiments, the crosslinked polyvinvlpyrrolidone thickening agent is present in an amount of from 3 wt % to 8 wt %, further optionally from 5 to 7 wt%, based on the weight of the composition.

In some embodiments, the compositions of the invention may optionally comprise an additional orally acceptable thickening agent, selected from one or more of, without limitation, carbomers, also known as carboxyvinyl polymers, carrageenans, also known as Irish moss and more particularly carrageenan (iota-carrageenan), high molecular weight polyethylene glycols (such as CARBOWAX®, available from The Dow Chemical Company), cellulosic polymers such as hydroxyethylcellulose, carboxymethylcellulose (CMC) and salts thereof, e.g., CMC sodium, natural gums such as karaya, xanthan, gum arabic and tragacanth, and colloidal magnesium aluminum silicate and mixtures of the same. Optionally, such additional thickening agents are present in a total amount of about 0.1 wt% to about 50 wt%, for example about 0.1 wt% to about 35 wt% or about 1 wt% to about 15 wt%, based on the weight of the composition.

In some embodiments, the composition further comprises polymer thickeners selected from (i) polyethylene glycol, (ii) polyethylene glycol - polypropylene glycol block co-polymers having a molecular weight of at least 5000, and (iii) combinations thereof.

In some embodiments, the composition comprises an ethylene oxide, propylene oxide block co-polymer of formula (ethylene oxide)ₓ-(propylene oxide)_{y} wherein x is an integer of 80-150, e.g. 100-130, e.g. about 118, and y is an integer 30-80, e.g. about 60-70, e.g. about 66, having an average molecular weight of greater than 5000, e.g., 8000 - 13000 Da, e.g. about 9800;

In some embodiments, the composition comprises an ethylene oxide, propylene oxide block co-polymer of average molecular weight greater than 5000 Da, being substantially free of an ethylene oxide, propylene oxide block co-polymer of average molecular weight less than 5000 Da. Optionally, the ethylene oxide, propylene oxide block co-polymer is present in an amount of from 5 wt % to 10 wt % based on the weight of the composition. Block copolymers of ethylene oxide / propylene oxide are useful, but higher molecular weight, e.g., > 5000Da are preferred, e.g. including PLURACARE® L1220 (available from BASF, Wyandotte, Mich., United States of America).

In some embodiments, the composition further comprises polyethylene glycol of average molecular weight 400 to 800 Da, e.g., about 600 Da. Low or medium molecular weight polyethylene glycol, e.g., PEG 400, PEG 600, PEG 800, PEG 1000 and mixtures thereof are useful in the compositions of some embodiments of the invention.

Further optionally, the polyethylene glycol may be present in an amount of from 5 wt % to 15 wt % based on the weight of the composition.

In some embodiments, the oral care compositions may additionally comprise a stabilizing amount of an additional linear polyvinylpyrrolidone.

The compositions of the invention may also comprise various dentifrice ingredients to adjust the rheology and feel of the composition such as humectants, surface active agents, or gelling agents, etc.

In some embodiments, the oral care composition comprises a vehicle for the active components. The vehicle may comprise humectants, e.g. selected from glycerin, propylene glycol or a combination thereof.

In some embodiments, the oral care composition comprises from about 35 to about 60 wt%, optionally from about 30 to about 55 wt% humectant based on the weight of the composition.

In some embodiments, the composition further comprises propylene glycol in an amount of from 15 wt % to 30 wt % based on the weight of the composition.

In some embodiments, the composition further comprises glycerin in an amount of from 15 wt % to 30 wt % based on the weight of the composition.

Typical compositions of the invention have a "low water" content, meaning that a total concentration of water, including any free water and all water contained in any ingredients, is less than about 5 wt%, preferably less than 3 wt%, preferably less than 2 wt% water.

Optionally, the composition contains less than 3 wt% water based on the weight of the composition. In some embodiments, the oral care composition contains less than 2 wt% water, e.g., less than 1 wt% water. In some embodiments, the composition is substantially anhydrous.

It is preferred that the vehicle ingredients in particular provide a dentifrice with a viscosity of about 10,000 CPS to about 700,000 CPS, preferably about 30,000 CPS to about 300,000 CPS.

As recognized by one of skill in the art, the oral compositions of the invention optionally include other materials, such as for example, anti-caries agents, desensitizing agents, viscosity modifiers, diluents, surface active agents, such as surfactants, emulsifiers, and foam modulators, pH modifying agents, abrasives, in addition to those listed above, humectants, mouth feel agents, sweetening agents, flavor agents, colorants, preservatives, and combinations thereof. It is understood that while general attributes of each of the above categories of materials may differ, there may be some common attributes and any given material may serve multiple purposes within two or more of such categories of materials. Preferably, the carrier is selected for compatibility with other ingredients of the composition.

Flavorants, sweeteners, colorants, foam modulators, mouth-feel agents and others additively may be included if desired, in the composition.

The compositions of the present invention may comprise a surface active agent (surfactant). Suitable surfactants include without limitation water-soluble salts of C₈₋₂₀ alkyl sulfates, sulfonated monoglycerides of C₈₋₂₀ fatty acids, sarcosinates, taurates, sodium lauryl sulfate, sodium cocoyl monoglyceride sulfonate, sodium lauryl sarcosinate, sodium lauryl isoethionate, sodium laureth carboxylate and sodium dodecyl benzenesulfonate, and cocoamidopropyl betaine.

In some embodiments, the composition may additionally comprise an anionic surfactant, e.g., sodium lauryl sulfate (SLS). In some embodiments, the composition further comprises an anionic surfactant in an amount of from 0.5 to 3 wt% based on the weight of the composition.

The compositions of the present invention optionally comprise one or more further active material(s), which is or are operable for the prevention or treatment of a condition or disorder of hard or soft tissue of the oral cavity, the prevention or treatment of a physiological disorder or condition, or to provide a cosmetic benefit.

In various embodiments of the present invention, the oral composition comprises an anticalculus (tartar control) agent. Generally, tartar control agents are categorized as being incompatible with some whitening agents, but embodiments of the present invention incorporate tartar control agents and whitening agents in a single phase whitening composition.

Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. Typically, the anticalculus agent is present at about 0.1% to about 30 wt% based on the weight of the composition.

The oral composition may include a mixture of different anticalculus agents.

In some embodiments, the composition additionally comprises a tartar control agent, e.g., selected from tetrasodium pyrophosphate (TSPP) and sodium tripolyphosphate (STPP).

In one preferred embodiment, tetrasodium pyrophosphate (TSPP) and sodium tripolyphosphate (STPP) are used. The anticalculus agent comprises TSPP at about 1-2% and STPP at about 7% to about 10%, each based on the weight of the composition.

The oral care composition can optionally include at least one orally acceptable source of fluoride ions. Any known or to be developed in the art may be used. Suitable sources of fluoride ions include fluoride, monofluorophosphate and fluorosilicate salts. One or more fluoride ion-releasing compound is optionally present in an amount providing a total of about 100 to about 20,000 ppm, about 200 to about 5,000 ppm, or about 500 to about 2,500 ppm, fluoride ions, each based on the weight of the composition.

The compositions may include a stannous ion or a stannous ion source. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01 % to about 10%, for example about 0.1% to about 7% or about 1% to about 5%, each based on the weight of the composition.

In some embodiments, the compositions of the invention optionally comprise an antimicrobial (e.g., antibacterial) agent, e.g., triclosan. A further illustrative list of useful antibacterial agents is provided in such as those listed in U.S. Pat. No. 5,776,435 to Gaffar et al., the contents of which are incorporated herein by reference. One or more antimicrobial agents are optionally present in an antimicrobial effective total amount, typically about 0.05% to about 10%, for example about 0.1% to about 3%, each based on the weight of the composition.

In some embodiments, the compositions of the invention optionally comprise an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

The compositions of the invention may optionally comprise a sialagogue or saliva-stimulating agent, an antiplaque agent, an anti-inflammatory agent, and/or a desensitizing agent.

While ingredients are sometimes identified herein by category, e.g., humectant, antioxidant, thickener, etc., this identification is for convenience and clarity, but is not intended to be limiting. All of the ingredients in the compositions may have functions in addition to their primary function, and may contribute to the overall properties of the composition, including its stability, efficacy, consistency, mouthfeel, taste, odor and so forth.

Methods are provided to whiten an oral surface in a human or animal subject comprising storing in stable form a composition of the invention, and contacting said composition with the oral surface. As used herein "animal subject" includes higher order non-human mammals such as canines, felines, and horses. The oral care composition is contacted with an oral surface of the mammalian subject to thereby whiten teeth in a highly efficacious manner, without any negative interaction between the whitening agent, the peroxide incompatible abrasive, and other ingredients.

In various embodiments, it is preferred that the oral care composition is applied and contacted with the oral surface. The dentifrice, prepared in accordance with particular embodiments of the invention, is preferably applied regularly to an oral surface, preferably on a daily basis, at least one time daily for multiple days, but alternately every second or third day. Preferably the oral composition is applied to the oral surfaces from 1 to 3 times daily, for at least 2 weeks up to 8 weeks, from four months to three years, or more up to lifetime.

The invention is illustrated in the following non-limiting examples.

### EXAMPLES

### Comparative Example 1

A dentifrice was prepared according to Comparative Example 1. The composition had the following ingredients as specified in Table 1, in which the amounts are in wt%:

**Table 1**

| **Ingredient** | **Comp. Ex. 1** | **Example 1** |
|---|---|---|
| PEG₁₁₈/PPG₆₆ co-polymer (Pluracare L1220F) | 7.5 | 7 |
| Glycerin | 25.36 | 25.36 |
| Propylene glycol | 25 | 25 |
| PEG 600 | 10 | 10 |
| Crosslinked PVP | 3.5 | 3.5 |
| Crosslinked PVP / H₂O₂ complex | 11 | 11 |
| Tetrasodium pyrophosphate (TSPP) | 2 | 2 |
| Sucralosc | 0.05 | 0.05 |
| Sodium saccharin | 0.6 | 0.6 |
| Sodium monofluorophosphate | 0.76 | 0.76 |
| Sodium lauryl sulfate | 2 | 2 |
| Butylated hydroxytolune (BHT) | 0.03 | 0.03 |
| 85wt% syrupy phosphoric acid | 0.2 | 0.2 |
| Flavor | 2 | 2 |
| Calcined alumina | 10 | -- |
| Stcaric acid coated calcined alumina | -- | 1 |
| **Total** | **100** | **100** |

The dentifrice of Comparative Example 1 comprised a peroxide whitening agent comprising a complex of crosslinked polyvinvlpyrrolidone and hydrogen peroxide. The hydrogen peroxide comprised 2 wt% of the total weight of the dentifrice. The dentifrice comprised a substantially anhydrous vehicle comprising PEG₁₁₈/PPG₆₆ co-polymer (Pluracare L1220F), glycerin, propylene glycol and PEG 600.

The abrasive comprised a calcined alumina available in commerce from Nabaltec AG, Germany, under the trade name NO 283.

The stability of the hydrogen peroxide in the dentifrice of Comparative Example 1 was evaluated using two different testing protocols. One testing protocol evaluated the chemical stability of hydrogen peroxide in the dentifrice while the other testing protocol evaluated the physical stability of the entire dentifrice formula.

Hydrogen peroxide chemically decomposes into the decomposition products of water and oxygen gas, with two moles of hydrogen peroxide producing one mole of oxygen. However, one mole of oxygen gas takes up more volume than 2 moles of hydrogen peroxide and so significant pressure can build up in a closed tube of dentifrice as hydrogen peroxide chemically degrades.

In Comparative Example 1, a conventional flexible polymer toothpaste tube having a shoulder width of 38 mm and a capacity of 125 g of dentifrice was filled with 125 g of the dentifrice and the tube was closed with a conventional closure.

In the first testing protocol to evaluate the chemical stability of hydrogen peroxide in the dentifrice, the progressive bloating of the hydrogen peroxide containing dentifrice formula of Comparative Example 1 resulting from the decomposition of the hydrogen peroxide was measured by taking a digital image of the side of the tube using a camera. Image analysis software was used to measure the area of the picture covered by the tube. As the tube swells due to internal pressure exerted by oxygen gas, the area of the picture covered by the tube correspondingly increases.

Images were taken the day after dentifrice preparation (as a baseline measurement), after three days of 55 °C accelerated aging, and after seven days of 55 °C accelerated aging. The resultant images were scaled to the width of the shoulder of the tube. Table 2 shows the increase in tube area as a function of time at 55 °C.

**Table 2**

| | 3 days 55 °C (% increase in area from baseline) | 7 days 55 °C (% increase in area from baseline) |
|---|---|---|
| Comparative Example 1 | 7.3 | 11.6 |
| Example 1 | 1.6 | 2.6 |

It may be seen that in Comparative Example 1 there was significant increase in the imaged area of the tube, indicating that there was significant hydrogen peroxide decomposition over the course of the accelerated aging test. Thus, the composition of Comparative Example 1 had poor chemical stability in the accelerated aging test.

Then the physical stability of the dentifrice of Comparative Example 1 comprising hydrogen peroxide and the calcined alumina abrasive was evaluated.

To evaluate the physical stability of the dentifrice network, a sample of the dentifrice of Comparative Example 1 was centrifuged at 2050 rpm using an analytical centrifuge (Lumisizer 110 from L.U.M. GmbH, Berlin). The Lumisizer centrifuge measures separation of product by measuring optical transmission through the centrifuge tube as a function of time. If the liquid phase of the dentifrice separates from the solid phase, the optical transmission in the tube increases.

A predetermined weight of the dentifrice of Comparative Example 1 was tested. Table 3 shows the time which was required to generate a preset amount of liquid in the tube which was determined as being representative of an unacceptable physical separation between the solid and liquid phases. The longer the time to generate the preset amount of liquid, more physically stable was the dentifrice formula.

**Table 3**

| | Time to Separation |
|---|---|
| Comparative Example 1 | 5 hrs 40 min |
| Example 1 | 7 hrs 10 min |

### Example 1

A dentifrice was prepared according to Example 1, the composition of which is also specified in Table 1.

The dentifrice of Example 1 comprised the same composition as the dentifrice of Example 1 except that in Example 1 the abrasive comprised calcined alumina pre-coated with stearic acid.

Again, the stability of the hydrogen peroxide in the dentifrice of Example 1 was evaluated using the same two testing protocols as described above for Comparative Example 1.

In the first testing protocol to evaluate the chemical stability of hydrogen peroxide in the dentifrice, again the same type of conventional flexible polymer toothpaste tube was filled with 125 g of the dentifrice of Example 1 and the tube was closed with a conventional closure.

Again, images were taken the day after dentifrice preparation (as a baseline measurement), after three days of 55 °C accelerated aging, and after seven days of 55 °C accelerated aging. The resultant images were scaled to the width of shoulder of the tube. Table 2 shows the increase in tube area as a function of time at 55 °C for the dentifrice of Example 1.

It may be seen from a comparison of the resultant data from Example 1 and Comparative Example 1 that the stearic acid coating on the calcined alumina abrasive particles significantly reduced decomposition of hydrogen peroxide in the dentifrice composition, thereby significantly enhancing the chemical stability of the peroxide whitening agent in the dentifrice.

In the second testing protocol to evaluate the physical stability of the dentifrice comprising hydrogen peroxide and a calcined alumina abrasive pre-coated with stearic acid, the same weight of the dentifrice was centrifuged and tested as described above for Comparative Example 1. The dentifrices of Example 1 and Comparative Example 1 had the same amount by weight of liquid and solid ingredients.

Table 3 shows the separation time to achieve the same degree of separation between the solid and liquid phases. It will be seem that the separation time for Example 1 was significantly higher than for Comparative Example 1.

In addition to improving the chemical stability of a peroxide containing dentifrice formula as shown in Table 2, Table 3 shows that coating of alumina with stearic acid also improved the physical stability of the network holding together the solid and liquid phases of the dentifrice.

These test results cumulatively show that pre-coating alumina with stearic acid improves the chemical stability of hydrogen peroxide in dentifrice and also improves the overall physical stability of the dentifrice network.

### Example 2

Example 1 demonstrates that the pre-coating of small particle calcined alumina with stearic acid improved the peroxide stability in a non-aqueous dentifrice formula as compared to the addition of uncoated alumina.

In Examples 2 and 3 and Comparative Example 2 a further laboratory experiment was conducted to demonstrate that the coating material must be compatible with hydrogen peroxide.

Example 2, like Example 1, tested stearic acid which is a saturated fatty acid consisting of an eighteen carbon chain attached to a carboxylic acid group.

To assess the performance of another fatty acid coating material, Example 3 tested oleic acid which is a monounsaturated fatty acid consisting of an eighteen carbon chain attached to a carboxylic acid group.

For comparison, Comparative Example 2 tested another polymer which could also attach to the surface of an alumina particle through carboxylic acid functionality, in particular a PVM/MA copolymer, sold under the commercial name Gantrez S-97 by ISP.

In each of Examples 2 and 3 and Comparative Example 2, a respective solution with the composition identified below in Table 4 was prepared.

**Table 4**

| | Example 2 (wt %) | Example 3 (wt %) | Comparative Example 2 (wt %) |
|---|---|---|---|
| Propylene glycol | 88.5 | 88.5 | 88.5 |
| Water | 5.0 | 5.0 | 5.0 |
| PVP-hydrogen peroxide | 5.5 | 5.5 | 5.5 |
| Stearic Acid | 1 | -- | -- |
| Oleic Acid | -- | 1 | -- |
| PVM/MA copolymer | -- | -- | 1 |

Each solution was prepared by dispersing 5.5 wt% PVP-hydrogen peroxide complex (to deliver 1 wt% hydrogen peroxide in the composition) in propylene glycol and water. After the hydrogen peroxide complex was dispersed, a sample of the respective coating material, in the form of a powder, was added to the aqueous dispersion, which was thereafter stirred vigorously for a period of 48 hours in order to disperse the coating material uniformly in aqueous solution.

In each composition, after complete dispersion of the coating material, the pH of the aqueous solution was adjusted to 7.2 using sodium hydroxide.

Two samples of each solution of Examples 2 and 3 and Comparative Example 2 were aliquoted into a respective separate container after preparation. One sample was analyzed for initial peroxide content and the other sample was aged at 55 °C for a period of 10 days in an accelerated aging test. After accelerated aging the level of peroxide remaining in the sample was measured for each solution of Examples 2 and 3 and Comparative Example 2. The results are shown in Table 5 (below).

**Table 5**

| | Example 2 | Example 3 | Comparative Example 2 |
|---|---|---|---|
| % H₂O₂ - Initial | 1.16 | 1.04 | 1.15 |
| % H₂O₂ - After 10 days at 55°C | 0.96 | 1.00 | 0.85 |
| % H₂O₂ remaining | 83 | 96 | 74 |

Table 5 shows that hydrogen peroxide is more stable in the presence of stearic acid and oleic acid than in the presence of PVM/MA copolymer. These results confirm that the chemical structure of a coating material is critical for providing hydrogen peroxide stability in a dentifrice formula, and that fatty acids according to the invention provide such hydrogen peroxide stability.

In summary, the data described in the Examples evidences the unexpected improvement in the chemical stability of the peroxide whitening agent and the unexpected improvement in the physical stability of the dentifrice incorporating such a peroxide whitening agent and an abrasive in the form of particles pre-coated with a coating layer in accordance with the invention.

While particular embodiments of the invention have been illustrated and described, it will be obvious to those skilled in the art that various changes and modifications may be made without departing from the scope of the invention as defined in the appended claims.

## Claims

1. An oral care composition comprising (i) a peroxide whitening agent, and (ii) an abrasive in the form of particles, wherein the particles are pre-coated with a coating layer composed of a coating material selected from at least one substantially saturated C₂ - C₃₀ fatty acid, at least one monounsaturated C₁₂ - C₃₀ fatty acid, and at least one silicone polymer, or any mixture of two or more thereof, wherein the substantially saturated C₂ - C₃₀ fatty acid and the monounsaturated C₁₂ - C₃₀ fatty acid each have a carbon-carbon bond saturation of at least 90%.

2. The composition of claim 1 wherein the coating material has an average molecular weight of less than 500.

3. The composition of claim 1 or claim 2 wherein the substantially saturated C₂ - C₃₀ fatty acid and the monounsaturated C₁₂ - C₃₀ fatty acid each have a carbon-carbon bond saturation of at least 94%.

4. The composition of any preceding claim wherein the substantially saturated C₂ - C₃₀ fatty acid comprises a substantially saturated C₁₂ - C₂₄ fatty acid, optionally,
wherein the substantially saturated C₁₂ - C₂₄ fatty acid comprises stearic acid.

5. The composition of any preceding claim wherein the monounsaturated C₁₂ - C₃₀ fatty acid comprises a monounsaturated C₁₆ - C₃₀ fatty acid, optionally,
wherein the monounsaturated C₁₆ - C₃₀ fatty acid comprises oleic acid.

6. The composition of any preceding claim wherein the silicone compound comprises a polydialkysiloxane, optionally,
wherein the silicone compound comprises polydimethylsiloxane.

7. The composition of any preceding claim wherein the coating layer is nonionic, optionally wherein the coating layer has a thickness of at least 1 angstrom, and further optionally,
wherein the coating layer comprises from 0.01 to 10 wt% of the weight of the pre-coated abrasive particles.

8. The composition of any preceding claim wherein the abrasive is selected from calcined alumina, silica, zirconium oxide, calcium pyrophosphate, dicalcium phosphate and precipitated calcium carbonate, or a combination of two or more thereof, and/or
wherein the pre-coated abrasive has an average particle size of from 1 to 10 microns, and/or
wherein the pre-coated abrasive is present in an amount of from 2 wt % to 20 wt % based on the weight of the composition.

9. The composition of any preceding claim wherein the peroxide whitening agent is selected from hydrogen peroxide, crosslinked polyvinylpyrrolidone complexed with hydrogen peroxide, urea peroxide, calcium peroxide and sodium percarbonate, and a combination of two or more thereof, optionally,
wherein the peroxide whitening agent comprises crosslinked polyvinvlpyrrolidone complexed with hydrogen peroxide, the abrasive comprises calcined alumina and the coating material comprises stearic acid, and further optionally,
wherein the crosslinked polyvinylpyrrolidone complexed with hydrogen peroxide is present in an amount of from 0.5 wt % to 16.5 wt % based on the weight of the composition.

10. The composition of any preceding claim wherein the whitening complex contains 10-30 wt% hydrogen peroxide and 5-15 wt% total nitrogen, based on the weight of the whitening complex, optionally,
wherein the total amount of hydrogen peroxide is from 0.1 wt% to 5 wt% based on the weight of the composition.

11. The composition of any preceding claim further comprising a humectant selected from glycerin and propylene glycol, and a combination thereof, optionally,
wherein the humectant is present in an amount of from 35 wt % to 60 wt % based on the weight of the composition, and further optionally,
wherein the humectant is present in an amount of from 30 wt % to 55 wt % based on the weight of the composition.

12. The composition of any preceding claim comprising propylene glycol in an amount of from 15 wt % to 30 wt % based on the weight of the composition, and/or
comprising glycerin in an amount of from 15 wt % to 30 wt % based on the weight of the composition.

13. The composition of any preceding claim which contains less than 3 wt% water based on the weight of the composition, and/or
which is a single phase composition.

14. The composition of any preceding claim, wherein the abrasive particles have an oil of absorption of from about 5g to about 250g/100g, and/or
wherein the abrasive particles have a surface area of from about 0.1 to 500 m²/g.

15. A method of tooth whitening comprising applying the composition of any one of claims 1 to 14 to the surface of a mammalian tooth.

## Patentansprüche

1. Mundpflegezusammensetzung, die (i) ein Peroxid-Weißungsmittel und (ii) einen Abrasivstoff in der Form von Teilchen umfasst, wobei die Teilchen mit einer Beschichtungsschicht vorbeschichtet sind, die sich aus einem Beschichtungsmaterial zusammensetzt, das aus mindestens einer im Wesentlichen gesättigten C₂-C₃₀-Fettsäure, mindestens einer einfach ungesättigten C₁₂-C₃₀-Fettsäure und mindestens einem Silikonpolymer oder einem beliebigen Gemisch von zwei oder mehr davon ausgewählt ist, wobei die im Wesentlichen gesättigte C₂-C₃₀-Fettsäure und die einfach ungesättigte C₁₂-C₃₀-Fettsäure jeweils eine Kohlenstoff-Kohlenstoff-Bindungssättigung von mindestens 90 % aufweisen.

2. Zusammensetzung nach Anspruch 1, wobei das Beschichtungsmaterial ein durchschnittliches Molekulargewicht von weniger als 500 aufweist.

3. Zusammensetzung nach Anspruch 1 oder 2, wobei die im Wesentlichen gesättigte C₂-C₃₀-Fettsäure und die einfach ungesättigte C₁₂-C₃₀-Fettsäure jeweils eine Kohlenstoff-Kohlenstoff-Bindungssättigung von mindestens 94 % aufweisen.

4. Zusammensetzung nach einem vorhergehenden Anspruch, wobei die im Wesentlichen gesättigte C₂-C₃₀-Fettsäure eine im Wesentlichen gesättigte C₁₂-C₂₄-Fettsäure umfasst, gegebenenfalls
wobei die im Wesentlichen gesättigte C₁₂-C₂₄-Fettsäure Stearinsäure umfasst.

5. Zusammensetzung nach einem vorhergehenden Anspruch, wobei die einfach ungesättigte C₁₂-C₃₀-Fettsäure eine einfach ungesättigte C₁₆-C₃₀-Fettsäure umfasst, gegebenenfalls
wobei die einfach ungesättigte C₁₆-C₃₀-Fettsäure Ölsäure umfasst.

6. Zusammensetzung nach einem vorhergehenden Anspruch, wobei die Silikonverbindung ein Polydialkylsiloxan umfasst, gegebenenfalls
wobei die Silikonverbindung Polydimethylsiloxan umfasst.

7. Zusammensetzung nach einem vorhergehenden Anspruch, wobei die Beschichtungsschicht nichtionisch ist, gegebenenfalls wobei die Beschichtungsschicht eine Dicke von mindestens einem Ängström aufweist und weiterhin gegebenenfalls
wobei die Beschichtungsschicht von 0,01 bis 10 Gew.-% des Gewichts der vorbeschichteten Abrasivstoffteilchen umfasst.

8. Zusammensetzung nach einem vorhergehenden Anspruch, wobei der Abrasivstoff aus kalziniertem Aluminiumoxid, Siliciumdioxid, Zirkonoxid, Calciumpyrophosphat, Dicalciumphosphat und gefälltem Calciumcarbonat oder einer Kombination von zwei oder mehr davon ausgewählt ist und/oder
wobei der vorbeschichtete Abrasivstoff eine durchschnittliche Teilchengröße von 1 bis 10 Mikron aufweist und/oder
wobei der vorbeschichtete Abrasivstoff in einer Menge von 2 Gew.-% bis 20 Gew.-%, bezogen auf das Gewicht der Zusammensetzung, vorliegt.

9. Zusammensetzung nach einem vorhergehenden Anspruch, wobei das Peroxid-Weißungsmittel aus Wasserstoffperoxid, vernetztem Polyvinylpyrrolidon, das mit Wasserstoffperoxid komplexiert ist, Harnstoffperoxid, Calciumperoxid und Natriumpercarbonat und einer Kombination von zwei oder mehr davon ausgewählt ist, gegebenenfalls
wobei das Peroxid-Weißungsmittel vernetztes Polyvinylpyrrolidon, das mit Wasserstoffperoxid komplexiert ist, umfasst, der Abrasivstoff kalziniertes Aluminiumoxid umfasst und das Beschichtungsmaterial Stearinsäure umfasst und weiterhin gegebenenfalls
wobei das vernetzte Polyvinylpyrrolidon, das mit Wasserstoffperoxid komplexiert ist, in einer Menge von 0,5 Gew.-% bis 16,5 Gew.-%, bezogen auf das Gewicht der Zusammensetzung, vorliegt.

10. Zusammensetzung nach einem vorhergehenden Anspruch, wobei der Weißungskomplex 10-30 Gew.-% Wasserstoffperoxid und 5-15 Gew.-% Gesamtstickstoff, bezogen auf das Gewicht des Weißungskomplexes, umfasst, gegebenenfalls
wobei die Gesamtmenge an Wasserstoffperoxid von 0,1 Gew.-% bis 5 Gew.-%, bezogen auf das Gewicht der Zusammensetzung, ausmacht.

11. Zusammensetzung nach einem vorhergehenden Anspruch, die weiterhin ein Feuchthaltemittel umfasst, das aus Glycerin und Propylenglykol und einer Kombination davon ausgewählt ist, gegebenenfalls
wobei das Feuchthaltemittel in einer Menge von 35 Gew.-% bis 60 Gew.-%, bezogen auf das Gewicht der Zusammensetzung, vorliegt und weiterhin gegebenenfalls
wobei das Feuchthaltemittel in einer Menge von 30 Gew.-% bis 55 Gew.-%, bezogen auf das Gewicht der Zusammensetzung, vorliegt.

12. Zusammensetzung nach einem vorhergehenden Anspruch, die Propylenglykol in einer Menge von 15 Gew.-% bis 30 Gew.-%, bezogen auf das Gewicht der Zusammensetzung, umfasst und/oder
die Glycerin in einer Menge von 15 Gew.-% bis 30 Gew.-%, bezogen auf das Gewicht der Zusammensetzung, umfasst.

13. Zusammensetzung nach einem vorhergehenden Anspruch, die weniger als 3 Gew.-% Wasser, bezogen auf das Gewicht der Zusammensetzung, umfasst und/oder
bei der es sich um eine einphasige Zusammensetzung handelt.

14. Zusammensetzung nach einem vorhergehenden Anspruch, wobei die Abrasivstoffteilchen ein Absorptionsöl von etwa 5 g bis etwa 250 g/100 g aufweisen und/oder
wobei die Abrasivstoffteilchen einen Oberflächenbereich von etwa 0,1 bis 500 m²/g aufweisen.

15. Verfahren zur Zahnweißung, das das Aufbringen der Zusammensetzung nach einem der Ansprüche 1 bis 14 auf die Oberfläche eines Zahns eines Säugers umfasst.

## Revendications

1. Une composition de soins buccaux comprenant (i) un agent de blanchiment peroxyde et (ii) un abrasif sous la forme de particules, dans laquelle les particules sont pré-revêtues avec une couche de revêtement composée d'un matériau de revêtement sélectionné parmi au moins un acide gras en C₂ - C₃₀ sensiblement saturé, au moins un acide gras en C₁₂ - C₃₀ mono-insaturé et au moins un polymère de silicone, ou un mélange de deux ou plus de deux de ces derniers, dans laquelle l'acide gras en C₂ - C₃₀ sensiblement saturé et l'acide gras en C₁₂ - C₃₀ mono-insaturé ont chacun une saturation de la liaison carbone - carbone d'au moins 90%.

2. La composition selon la revendication 1 dans laquelle le matériau de revêtement a un poids moléculaire moyen inférieur à 500.

3. La composition selon la revendication 1 ou la revendication 2 dans laquelle l'acide gras en C₂ - C₃₀ sensiblement saturé et l'acide gras en C₁₂ - C₃₀ mono-insaturé ont chacun une saturation de la liaison carbone - carbone d'au moins 94 %.

4. La composition selon l'une quelconque des revendications précédentes dans laquelle l'acide gras en C₂ - C₃₀ sensiblement saturé comprend un acide gras en C₁₂ - C₂₄ sensiblement saturé,
en option, dans laquelle l'acide gras en C₁₂ - C₂₄ sensiblement saturé comprend de l'acide stéarique.

5. La composition selon l'une quelconque des revendications précédentes dans laquelle l'acide gras en C₁₂ - C₃₀ mono-insaturé comprend un acide gras en C₁₆ - C₃₀ mono-insaturé,
en option, dans laquelle l'acide gras en C₁₆ - C₃₀ mono-insaturé comprend de l'acide oléique.

6. La composition selon l'une quelconque des revendications précédentes dans laquelle le composé de silicone comprend un polydialkysiloxane,
en option, dans laquelle le composé de silicone comprend du polydiméthylsiloxane.

7. La composition selon l'une quelconque des revendications précédentes dans laquelle la couche de revêtement est non ionique,
en option, dans laquelle la couche de revêtement a une épaisseur d'au moins 1 angström, et
en option en outre, dans laquelle la couche de revêtement comprend entre 0,01 et 10 % en poids du poids des particules abrasives pré-revêtues.

8. La composition selon l'une quelconque des revendications précédentes dans laquelle l'abrasif est sélectionné parmi l'alumine calcinée, la silice, l'oxyde de zirconium, le pyrophosphate de calcium, le phosphate de dicalcium et le carbonate de calcium précipité, ou une combinaison de deux ou plus de deux de ces derniers, et / ou
dans laquelle l'abrasif pré-revêtu a une taille de particule moyenne comprise entre 1 et 10 microns, et / ou
dans laquelle l'abrasif pré-revêtu est présent dans une quantité comprise entre 2 % en poids et 20 % en poids sur la base du poids de la composition.

9. La composition selon l'une quelconque des revendications précédentes dans laquelle l'agent de blanchiment peroxyde est sélectionné parmi le peroxyde d'hydrogène, la polyvinylpyrrolidone réticulée complexée avec le peroxyde d'hydrogène, le peroxyde d'urée, le peroxyde de calcium et le percarbonate de sodium, et une combinaison de deux ou plus de deux de ces derniers,
en option, dans laquelle l'agent de blanchiment peroxyde comprend de la polyvinylpyrrolidone réticulée complexée avec le peroxyde d'hydrogène, l'abrasif comprend de l'alumine calcinée et le matériau de revêtement comprend de l'acide stéarique, et
en option en outre, dans laquelle la polyvinylpyrrolidone réticulée complexée avec le peroxyde d'hydrogène est présente dans une quantité comprise entre 0,5 % en poids et 16,5 % en poids sur la base du poids de la composition.

10. La composition selon l'une quelconque des revendications précédentes dans laquelle le complexe de blanchiment contient entre 10 et 30 % en poids de peroxyde d'hydrogène et entre 5 et 15 % en poids d'azote total, sur la base du poids du complexe de blanchiment,
en option, dans laquelle la quantité totale de peroxyde d'hydrogène est comprise entre 0,1 % en poids et 5 % en poids sur la base du poids de la composition.

11. La composition selon l'une quelconque des revendications précédentes comprenant en outre un humidifiant sélectionné parmi la glycérine et le propylène glycol, et une combinaison de ces derniers,
en option en outre dans laquelle l'humidifiant est présent dans une quantité comprise entre 35 % en poids et 60 % en poids sur la base du poids de la composition, et
en option en outre, dans laquelle l'humidifiant est présent dans une quantité comprise entre 30 % en poids et 55 % en poids sur la base du poids de la composition.

12. La composition selon l'une quelconque des revendications précédentes comprenant du propylène glycol dans une quantité comprise entre 15 % en poids et 30 % en poids sur la base du poids de la composition, et / ou
comprenant de la glycérine dans une quantité comprise entre 15 % en poids et 30 % en poids sur la base du poids de la composition.

13. La composition selon l'une quelconque des revendications précédentes laquelle contient moins de 3 % en poids d'eau sur la base du poids de la composition, et / ou
qui est une composition à phase unique.

14. La composition selon l'une quelconque des revendications précédentes, dans laquelle les particules abrasives ont une huile d'absorption comprise entre environ 5 g et environ 250 g / 100 g et / ou
dans laquelle les particules abrasives ont une superficie comprise entre environ 0,1 et 500 m²/g.

15. Un procédé de blanchiment de dent consistant à appliquer la composition de l'une quelconque des revendications 1 à 14 sur la surface d'une dent de mammifère.
